(19) Europäisches Patentamt
European Patent Office
Office européen des brevets

(11) **EP 3 285 696 B1**

(12) **EUROPÄISCHE PATENTSCHRIFT**

(45) Veröffentlichungstag und Bekanntmachung des Hinweises auf die Patenterteilung:
**25.05.2022   Patentblatt 2022/21**

(21) Anmeldenummer: **16716578.6**

(22) Anmeldetag: **15.04.2016**

(51) Internationale Patentklassifikation (IPC):
*A61F 2/64* (2006.01)      *A61F 2/66* (2006.01)
*A61F 2/68* (2006.01)

(52) Gemeinsame Patentklassifikation (CPC):
**A61F 2/64; A61F 2/6607; A61F 2/70; A61F 2/74; A61F 5/0125;** A61F 2002/5006; A61F 2002/5033; A61F 2002/6818; A61F 2002/701; A61F 2002/704; A61F 2002/705; A61F 2002/7625; A61F 2002/7635; A61F 2002/764; A61F 2005/0188

(86) Internationale Anmeldenummer:
**PCT/EP2016/058356**

(87) Internationale Veröffentlichungsnummer:
**WO 2016/169850 (27.10.2016 Gazette 2016/43)**

(54) **VERFAHREN ZUR STEUERUNG EINER DÄMPFUNGSVERÄNDERUNG BEI EINEM KÜNSTLICHEN GELENK**

METHOD FOR CONTROLLING A CHANGE OF DAMPING IN AN ARTIFICIAL JOINT

PROCÉDÉ POUR CONTRÔLER UN CHANGEMENT D'AMORTISSEMENT D'UNE PROTHÈSE ARTICULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2015   DE 102015106384**

(43) Veröffentlichungstag der Anmeldung:
**28.02.2018   Patentblatt 2018/09**

(73) Patentinhaber: **Otto Bock Healthcare Products GmbH**
**1110 Wien (AT)**

(72) Erfinder: **SEIFERT, Dirk**
**1070 Wien (AT)**

(74) Vertreter: **Gramm, Lins & Partner**
**Patent- und Rechtsanwälte PartGmbB**
**Theodor-Heuss-Straße 1**
**38122 Braunschweig (DE)**

(56) Entgegenhaltungen:
EP-A2- 1 447 062      WO-A1-2011/057793
DE-A1-102012 003 369   US-A1- 2003 125 814

**Beschreibung**

Die Erfindung betrifft ein Verfahren zur Steuerung einer Widerstandsänderung bei einem künstlichen Gelenk einer Orthese, eines Exoskelettes oder Prothese einer unteren Extremität, wobei das künstliche Gelenk ein Oberteil und ein Unterteil aufweist, die um eine Schwenkachse schwenkbar aneinander befestigt sind, wobei eine Widerstandseinheit zwischen dem Oberteil und dem Unterteil befestigt ist, um einen Widerstand gegen eine Einbeugung oder Streckung des künstlichen Gelenks bereitzustellen und der Widerstandseinheit eine Verstelleinrichtung zugeordnet ist, über die der Widerstand verändert wird, wenn ein Sensorsignal einer der Verstelleinrichtung zugeordneten Steuereinheit die Verstelleinrichtung aktiviert. Das Verfahren wird insbesondere zur Steuerung des Bewegungs- oder Dämpfungsverhaltens künstlicher Kniegelenke eingesetzt, ist jedoch nicht darauf beschränkt, sondern kann auch für Hüft- oder Knöchelgelenke eingesetzt werden.

Künstliche Gelenke für Orthesen, Exoskelette oder Prothesen weisen ein Oberteil mit einem oberen Anschlussteil und ein Unterteil mit einem unteren Anschlussteil auf, die gelenkig miteinander verbunden sind. An dem oberen Anschlussteil sind bei künstlichen Kniegelenke in der Regel Aufnahmen für einen Oberschenkelstumpf oder eine Oberschenkelschiene angeordnet, während an dem unteren Anschlussteil ein Unterschenkelrohr oder eine Unterschenkelschiene mit einem Prothesenfuß oder einem Fußteil angeordnet sind. Bei einem noch vorhandenen Unterschenkel ist das Oberteil einer Prothese an einem Unterschenkelschaft angeordnet, an dem Unterteil ist der Prothesenfuß befestigt, bei Orthesen sind die jeweiligen Komponenten an den zugehörigen Gliedmaßen befestigt. Im einfachsten Fall sind das Oberteil und das Unterteil durch ein einachsiges Gelenk verschwenkbar miteinander verbunden.

Um unterschiedliche Anforderungen während der verschiedenen Phasen eines Schrittes oder bei anderen Bewegungen oder Verrichtungen möglichst natürlich erfüllen zu können oder zu unterstützen, ist häufig eine Widerstandseinrichtung vorgesehen, die einen Flexionswiderstand und einen Extensionswiderstand bereitstellt. Über den Flexionswiderstand wird eingestellt, wie leicht sich das Unterteil im Verhältnis zum Oberteil bei einer aufgebrachten Kraft verschwenken lässt. Der Extensionswiderstand bremst bei einem Kniegelenk die Vorwärtsbewegung des Unterteils und bildet unter anderem einen Streckanschlag aus, der Flexionswiderstand verhindert ein ungewolltes Einbeugen und begrenzt die maximale Einbeugung in der Schwungphase.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssenor, ein Neigungssenor und/oder ein Kraftsensor. In Abhängigkeit von den Sensordaten wird die Position des Extensionsanschlages ermittelt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2009 052 887 A1 beschreibt unter anderem ein Verfahren zur Steuerung eines orthetischen oder prothetischen Gelenkes mit einer Widerstandseinrichtung und Sensoren, wobei über Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beuge- und/oder Streckwiderstandes zugrunde gelegt wird.

Zur Steuerung der Veränderung des Dämpfungsverhaltens werden gemäß dem Stand der Technik die Sensordaten quantitativ ausgewertet, das heißt, dass in der Regel bestimmte Grenzwerte vorgegeben werden, bei deren Erreichen oder Nichterreichen der Aktuator aktiviert oder deaktiviert wird, so dass die Widerstandseinrichtung einen erhöhten oder verringerten Flexions- oder Extensionswiderstand bereitstellt.

Patienten können Prothesen, Exoskelette oder Orthesen in verschiedenen Umgebungen einsetzen. Sie können Treppen abwärts gehen, Rampen abwärts gehen oder mit verschiedenen Geschwindigkeiten in der Ebene gehen. Weiterhin können Lasten getragen werden, was sich ebenfalls auf das Verhalten der Prothese oder Orthese auswirkt. Insbesondere nach Beendigung der Schwungphase, also nach dem Aufsetzen des versorgten Beines, wenn das Körpergewicht auf das versorgte Bein verlagert wird, besteht für die Patienten vielfach das Bedürfnis nach erhöhter Sicherheit. Eine zu hohe initiale Flexionsdämpfung, also eine Dämpfung, die einer Einbeugung des künstlichen Kniegelenkes entgegenwirkt, würde aber zu einer stoßartigen Belastung in dem Hüftgelenk führen, was eine Verringerung des Tragekomforts und der Akzeptanz der Prothese oder Orthese zur Folge hätte.

Moderne computergesteuerte Dämpfungseinrichtungen sind in der Lage, sehr präzise und schnell den Widerstand gegen eine Flexion oder Extension anzupassen. Limitierend sind die Genauigkeit der ermittelten oder erfassten Daten, die Komplexität der zu verarbeitenden Informationen, die Zuverlässigkeit der Detektion der jeweils ausgeführten Bewegung und der konstruktive Aufwand.

Die DE 10 2012 003 369 A1 betrifft ein Verfahren zur Steuerung eines künstlichen Orthesen- oder Prothesenkniegelenkes, an dem eine Unterschenkelkomponente und eine Widerstandseinrichtung angeordnet sind, wobei die Widerstandseinrichtung mit zumindest einem Aktuator versehen ist. Über den Aktuator wird der Beugewiderstand in Abhängigkeit von Sensordaten verändert, die während der Benutzung des Orthesen- oder Prothesenkniegelenkes über einen Sensor ermittelt werden. Zur Steuerung des Widerstandes wird ausschließlich der Absolutwinkel der Unterschenkelkomponente über zumindest einen Inertialwinkelsensor ermittelt. Der ermittelte Absolutwinkel wird mit zumindest ein Schwellwert verglichen. Bei Erreichen eines Schwellwertes würde eine Veränderung des Beugewiderstandes initiiert.

Aufgabe der vorliegenden Erfindung ist es, ein Verfahren zur Steuerung eines künstlichen Gelenkes einer unteren Extremität, insbesondere eines künstlichen Kniegelenkes, bereitzustellen, mit dem eine zuverlässige, schnelle und kostengünstige Anpassung an unterschiedliche Gangsituationen und ein komfortables Gangverhalten bei gleichzeitiger maximaler Sicherheit erreicht werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das erfindungsgemäße Verfahren zur Steuerung einer Dämpfungsveränderung bei einem künstlichen Gelenk einer Orthese, eines Exoskelettes oder Prothese einer unteren Extremität, wobei das künstliche Gelenk ein Oberteil und ein Unterteil aufweist, die um eine Schwenkachse schwenkbar aneinander befestigt sind, wobei eine Widerstandseinheit zwischen dem Oberteil und dem Unterteil befestigt ist, um einen Widerstand gegen eine Einbeugung oder Streckung des künstlichen Gelenks bereitzustellen und der Widerstandseinheit eine Verstelleinrichtung zugeordnet ist, über die der Widerstand verändert wird, wenn ein Sensorsignal einer der Verstelleinrichtung zugeordneten Steuereinheit die Verstelleinrichtung aktiviert, sieht vor, dass der Widerstand in Abhängigkeit von der Lage und/oder Länge der Beinsehne und/oder deren zeitlichen Ableitungen verändert wird. Die Beinsehne wird somit als Steuerparameter oder Hilfsvariable eingesetzt, um auf Grundlage der Lage oder Länge oder einer Kombination aus Lage und Länge der Beinsehne zu erkennen, welche Bewegung gerade ausgeführt wird, so dass die notwendigen Widerstandsänderungen für die jeweilige Gangsituation oder anderen Aktivitäten wie Hinsetzen, Aufstehen, Fahrradfahren, Rückwärtsgehen oder Ähnliches passend eingeleitet werden kann. Die Lage der Beinsehne liefert zuverlässig Informationen über die Orientierung des Beines, unabhängig von einer Einbeugung, beispielsweise einer Standphasenflexion oder Standphasenextension. Weiterhin lässt sich aus der Lage der Beinsehne und einer Verkürzung der Beinsehne ohne Lageänderung auf ein stationäres Einsinken schließen, eine Vorwärtsrotation der Beinsehne zeigt ein Rampenabwärtsgehen an, eine Rückwärtsrotation beispielsweise ein Hinsetzen. Auf der Grundlage der Auswertung dieser Informationen wird durch die Steuereinheit die Verstelleinrichtung aktiviert, um den Widerstand in der Widerstandseinheit an die jeweilige Gangsituation anzupassen. Dazu werden Flexions- und Extensionswiderstand passend erhöht oder verringert. Die Länge der Beinsehne ergibt zudem Aufschluss über den Bewegungsfortschritt, verkürzt sich die Beinsehne beispielsweise bei einer Rückwärtsrotation, kann darüber der Fortschritt einer Hinsetzbewegung erfasst oder zumindest abgeschätzt werden. Dementsprechend erfolgt eine Anpassung des Widerstandes auf der Grundlage dieser Informationen. Ebenfalls relevant sind die zeitlichen Ableitungen hinsichtlich der Lage oder Länge der Beinsehne. Es werden die Geschwindigkeiten oder Beschleunigungen der jeweiligen Bewegung ermittelt, was beispielsweise Hinweise auf eine Gehgeschwindigkeit liefert, so dass dementsprechend geänderte Widerstandswerte in den jeweiligen Bewegungsphasen bereitgestellt werden. Das Verfahren ist nicht nur auf die Steuerung von Widerstandseinheiten in künstlichen Kniegelenken ausgerichtet, vielmehr können auch bei gedämpften Knöchelgelenken eine entsprechende Widerstandseinheit in Abhängigkeit von der Länge und/oder Lage der Beinsehne oder deren zeitlichen Ableitungen verändert werden. Gleiches gilt auch für eine Widerstandseinheit in einem Hüftgelenk. Das Verfahren kann bei einer Orthese, Prothese oder einer Sonderform der Orthese, nämlich einem Exoskelett, angewendet werden.

Als Beinsehne wird die Verbindungslinie zwischen einem Hüftdrehpunkt und einem Fußpunkt verwendet. Der Hüftdrehpunkt wird beispielsweise bei einem Prothesenkniegelenk über einen Orthopädietechniker ermittelt. Der Hüftdrehpunkt legt auch die Segmentlänge des Oberschenkels fest, die als Abstand zwischen der Schwenkachse oder Knieachse und dem Hüftdrehpunkt definiert ist. Zwischen der Knieachse und einem Fußpunkt ist die Unterschenkellänge definiert. Als Fußpunkt kann entweder die Fußmitte, der Momentanpol einer Abrollbewegung oder der Endpunkt der Lotlinie des Unterschenkels auf dem Sohlenniveau des Fußteils, des Prothesenfußes oder auf dem Boden definiert werden. Bei Orthesen oder Exoskeletten ist ein Fußteil zur Auflage eines noch vorhandenen natürlichen Fußes nicht unbedingt notwendig, wenn die Steuerung einer Widerstandseinheit zwischen einem Oberschenkelteil und einem Unterschenkelteil ausgeführt werden soll.

Die Lage der Beinsehne oder auch der Beinsehnenwinkel kann als Summe aus einem ermittelten Unterschenkelwinkel und einem mit einem Faktor versehenen Kniewinkel abgeschätzt werden. Der Faktor liegt in einem Bereich zwischen 0,4 und 0,6, vorteilhafterweise ist die Lage der Beinsehne zur Vertikalen abgeschätzt die Summe aus dem Unterschenkelwinkel zur Vertikalen und dem halbierten Kniewinkel. Alternativ kann die Lage der Beinsehne über den Unterschenkelwinkel, den Kniewinkel und die Oberschenkelsegmentlänge mit der Unterschenkelsegmentlänge errechnet werden. Die jeweiligen Segmentlängen sind bekannt und in der Steuerungseinrichtung hinterlegt. Der Unterschenkelwinkel kann über Lagesensoren ermittelte werden, der Kniewinkel, der die relative Verschwenkung um die Knieachse anzeigt, wird als Winkel zwischen der Verlängerung der Längserstreckung des Unterschenkels zu der Längserstreckung des Oberschenkels definiert, die Messung kann über einen Winkelsensor erfolgen.

Der Unterschenkelwinkel und/oder der Oberschenkelwinkel können über einen Inertialwinkelsensor direkt gemessen werden. Alternativ kann ein Lagesensor an dem jeweiligen anderen Segment angeordnet sein, wobei über einen Kniewinkelsensor der Kniewinkel ermittelt und aus einer Kombination des Inertialwinkels des Oberschenkels mit dem Kniewinkel der Unterschenkelwinkel oder aus einer Kombination eines Lagesensors an dem Unterschenkel und dem Kniewinkelsensor der Oberschenkelwinkel ermittelt wird.

Die Länge der Beinsehne kann aus dem Kniewinkel und den Segmentlängen von Oberschenkel und Unterschenkel bestimmt werden. Über die Länge der Beinsehne oder deren Veränderung über die Zeit ergeben sich Rückschlüsse auf Bewegungsgeschwindigkeiten, in deren Abhängigkeit die Widerstände in der Dämpfungseinheit verändert werden.

Der Widerstand in der Widerstandseinheit kann auch in Abhängigkeit von der Richtung der Veränderung der Lage und/oder Länge der Beinsehne verändert werden. So kann eine Vorwärtsrotation durch eine Verringerung des Beinsehnenwinkels in Richtung auf eine Vertikale hin oder eine Vergrößerung des Beinsehnenwinkels von der Vertikalen weg als nachgebender Schritt beim Rampenabwärtsgehen detektiert werden, insbesondere, wenn die Beinsehnenlänge sich verkürzt. Aus der Verkürzung oder Verlängerung der Beinsehne ergibt sich ein Hinweis, ob eine Aufstehbewegung oder eine Hinsetzbewegung, ein Einsinken oder Aufstehen oder ein Treppab- oder Treppaufgehen ausgeführt wird.

Beim Vorwärtsgehen, also bei allen Gangsituationen, in denen eine Vorwärtsprogression stattfindet, rollt das Bein nach vorne ab. Die Beinorientierung, also die Orientierung der Beinsehne und deren Änderung können somit als Fortschrittsparameter für einen Schritt betrachtet werden. Nach dem Fersenkontakt oder Heel Strike weist das Bein meist eine Rückwärtsneigung auf, das heißt, dass die Beinsehne zur Vertikalen entgegen der Gangrichtung nach hinten geneigt ist. Anschließend rollt das Bein nach vorne ab, der Beinsehnenwinkel verringert sich in Richtung auf die Vertikale und vergrößert sich dann von der Vertikalen, bis am Ende der Standphase eine maximale Vorwärtsneigung vorliegt. Um eine Unterscheidung der Gangsituation zu präzisieren, wird vorteilhafterweise ein Quotient aus der Veränderung der Lage der Beinsehne und der Veränderung des Oberschenkelwinkels oder Unterschenkelwinkels ermittelt und zur Beurteilung der Gangsituation verwendet. Der Oberschenkelwinkel oder Unterschenkelwinkel oder deren zeitliche Ableitungen korrelieren mit der Bewegung des Beines während des Schrittes. Beim Vorwärtsgehen in der Ebene rollen der Oberschenkel und der Unterschenkel ebenfalls nach vorne ab, dies tritt sowohl mit als auch ohne Standphasenbeugung ein. Beim Abwärtsgehen auf Rampen bleibt der Oberschenkelwinkel annähernd konstant, beim Treppabgehen kippt der Oberschenkel nach hinten ab, der Winkel vergrößert sich somit relativ zur Vertikalen. Je nach ermittelter Gangsituation wird dann der Widerstand angepasst, beispielsweise die Flexionsdämpfung erhöht oder für einen bestimmten Winkelbereich verringert, um ein Einbeugen beim alternierenden Treppabgehen zu ermöglichen.

Als Parameter zur Erkennung der Gangsituation kann insbesondere ein Quotient aus der Änderung der Lage der Beinsehne und der Änderung des Oberschenkelwinkels oder des Unterschenkelwinkels ermittelt und verwendet werden. Insbesondere kann in Abhängigkeit von der Steigung des Graphen des jeweiligen Phasendiagrammes der Widerstand verändert werden. Wird der Oberschenkelwinkel oder Unterschenkelwinkel über die Beinsehnenlage oder den Beinsehnenwinkel aufgetragen, lassen sich Gangsituationen anhand der Steigung in dem Phasendiagramm unterscheiden. Die Steigung kann als oder aus einem Differenzenquotient $\Delta\varphi_i / \Delta\varphi_j$ oder dem Differentialquotienten $d\varphi_i / d\varphi_j$ des funktionellen Zusammenhangs zweier Winkel $\varphi_i$ und $\varphi_j$ bestimmt werden, wobei beispielsweise die momentane Tangente oder aber die Sekante über einen längeren Zeitraum herangezogen werden kann. Das Verhalten der Widerstandseinheit kann in Abhängigkeit von der Steigung an die entsprechende Situationen angepasst werden.

Neben der Veränderung der Widerstandseinheit bzw. des Widerstandes auf der Grundlage der Steigung im Phasendiagramm der Winkel, ist ebenfalls vorgesehen, dass diese Veränderung des Widerstandes auch auf Grundlage_eines Quotienten aus deren zeitlichen Ableitungen erfolgt, nämlich aus dem Quotienten der Änderung der Beinsehnengeschwindigkeit und der Änderung der Oberschenkelgeschwindigkeit oder Unterschenkelgeschwindigkeit.

Eine weitere Präzisierung der Unterscheidung der verschiedenen Gangsituationen kann dadurch erfolgen, dass zur Detektion der Standphase oder des Stehens ein Kraftsensor eingesetzt wird, der eine auf das Unterteil einwirkende Axialkraft oder ein auf das Unterteil einwirkendes Moment erfasst. Ist das Unterteil unbelastet oder im Wesentlichen unbelastet, kann davon ausgegangen werden, dass sich die untere Extremität in der Schwungphase, Anhebephase oder Aufsetzphase befindet, was eine andere Einstellung der Widerstände erfordert als das Stehen oder die Standphase beim Gehen. Ein entsprechender Sensor kann hierzu die zusätzliche Information bereitstellen.

Vorteilhafterweise wird der Widerstand zusätzlich in Abhängigkeit von der Lage oder der Lageänderung des Oberteils und / oder des Unterteils verändert. Oberteil und Unterteil wirken vorteilhafterweise als Oberschenkel und Unterschenkel. Vorzugsweise wird der Widerstand insbesondere geändert, wenn die Lage und / oder die Lageänderung der Beinsehne einen vorbestimmten Grenzwert überschreitet oder unterschreitet.

Natürlich kann die Lage in Abhängigkeit aller dieser genannten Parameter oder nur einzelner der genannten Parameter geändert werden.

Die Widerstandseinheit kann beispielsweise als Aktuator, beispielsweise als hydraulische, pneumatische, magnetorheologische, magnetische, elektrische, mechanische oder elektromagnetische Widerstandseinheit ausgestaltet sein. Bei hydraulischen oder pneumatischen Widerstandseinheiten werden Überströmkanäle geschlossen, so dass diese Überströmkanäle kein Medium mehr aus einer Extensionskammer in eine Flexionskammer strömen kann. Auf diese Weise kann der Fluss des Mediums zwischen der Extensionskammer und der Flexionskammer ggf. auch vollständig unterbunden werden. Bei mechanischen Widerstandseinrichtungen wird beispielsweise die Reibung soweit erhöht, dass keine weitere Flexion stattfinden kann. Gleiches gilt für elektrisch aktuierte Widerstandseinheiten.

Es können auch Aktuatoren zum Einsatz kommen, die sowohl Energie aktiv in das System einbringen, als auch umgekehrt dem System Energie entziehen und auf diese Weise als Widerstandseinheit wirken. Aktuatoren können beispielsweise als Elektromotoren, hydraulische oder pneumatische Pumpen oder piezoelektrische Elemente ausgebildet sein.

Nachfolgend werden Ausführungsbeispiele der Erfindung anhand der beigefügten Figuren näher erläutert. Es zeigen:

Figur 1 - eine schematische Darstellung einer Beinprothese,

Figur 2 - eine schematische Darstellung einer Knieprothese mit Winkeln;

Figur 3 - eine Darstellung gemäß Figur 2 mit einer Parameterzuordnung;

Figuren 4a- 4c - Darstellungen unterschiedlicher Situationen beim Gehen; sowie

Figur 5 - verschiedene Darstellungen eines Oberschenkelwinkels über der Beinorientierung.

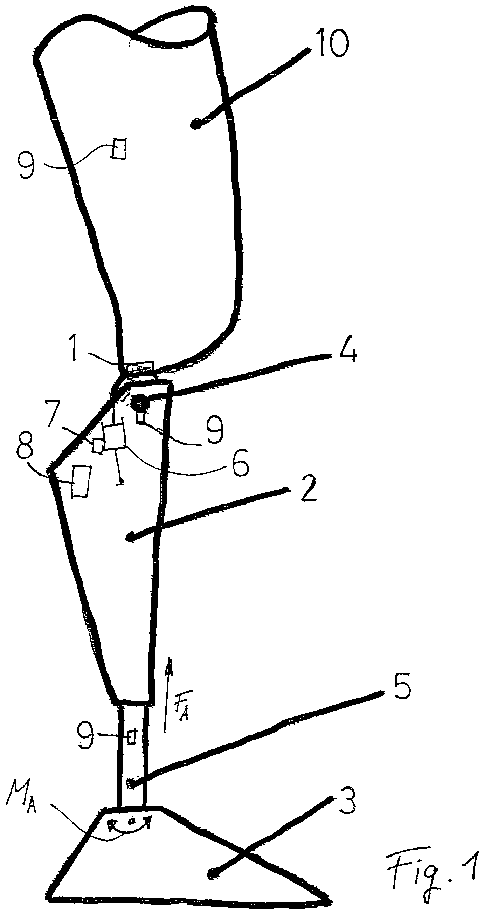

In der Figur 1 ist in einer schematischen Darstellung eine Beinprothese mit einem Oberteil 1 abgebildet, an dem ein Oberschenkelschaft 10 zur Aufnahme eines Oberschenkelstumpfes befestigt ist. An dem Oberteil 1 ist ein Unterteil 2 in Gestalt eines Unterschenkelteils schwenkbar angeordnet. Das Unterteil 2 ist an dem Oberteil 1 um eine Schwenkachse 4 schwenkbar gelagert. Das Unterteil 2 weist ein Unterschenkelrohr 5 auf, an dessen distalen Ende ein Prothesenfuß 3 befestigt ist, in dem eine Einrichtung zur Ermittlung der wirksamen Axialkraft auf das Unterschenkelrohr 5 sowie des Knöchelmomentes, das um die Befestigungsstelle des Prothesenfußes 3 an dem Unterschenkelrohr 5 wirksam ist, untergebracht sein kann.

In oder an dem Unterteil 2 ist eine Widerstandseinrichtung 6, die beispielsweise als Dämpfer oder Aktuator ausgebildet sein kann, angeordnet, die sich zwischen dem Oberteil 1 und dem Unterteil 2 abstützt, um einen einstellbaren Extensionswiderstand und Flexionswiderstand bereitzustellen. Der Widerstandseinrichtung 6 ist eine Verstelleinrichtung 7 zugeordnet, beispielsweise ein Motor, ein Magnet oder ein anderer Aktuator, über den der jeweilige Widerstand R innerhalb der Widerstandseinheit 6 verändert werden kann. Ist die Widerstandseinheit 6 als Hydraulikdämpfer oder Pneumatikdämpfer ausgebildet, kann über die Verstelleinrichtung 7 der jeweilige Strömungsquerschnitt eines Überströmkanals vergrößert oder verkleinert werden. Ebenso kann durch die Verstelleinrichtung 7 der Strömungswiderstand auf eine andere Art und Weise variiert werden. Dies kann beispielsweise durch Öffnen oder Schließen von Ventilen oder Veränderungen von Viskositäten oder magnetorheologischer Eigenschaften geschehen. Ist die Widerstandseinheit als ein Elektromotor im Generatorbetrieb ausgebildet, kann durch Veränderung des elektrischen Widerstandes eine Vergrößerung oder Verringerung der jeweiligen Widerstände gegen eine Flexion oder Extension eingestellt werden. Die Widerstandseinheit kann auch als mechanischer Widerstand gegen eine Flexion oder Extension ausgebildet sein, als Reibbremse oder als Elastomerelement mit veränderlichem Verformungswiderstand oder ein magnetorheologischer Dämpfer.

Um die Verstelleinrichtung 7 aktivieren oder deaktivieren zu können, ist eine Steuerungseinrichtung 8 dem Unterteil 2 zugeordnet, insbesondere in einer Unterschenkelverkleidung angebracht, über die ein entsprechendes Aktivierungs- oder Deaktivierungssignal an die Verstelleinrichtung 7 abgegeben wird. Die Verstelleinrichtung 7 wird auf der Basis von Sensordaten aktiviert oder deaktiviert, die Sensordaten werden von einem oder mehreren Sensoren 9 geliefert, die an dem künstlichen Kniegelenk angeordnet sind. Dies können Winkelsensoren, Inertialwinkelsensoren, Beschleunigungssensoren und/oder Kraftsensoren sein. Die Sensoren 9 sind mit der Steuereinrichtung 8 verbunden, beispielsweise per Kabel oder über eine drahtlose Sendeeinrichtung. In dem dargestellten Ausführungsbeispiel ist der Sensor 9 unter anderem als Kniewinkelsensor oder Inertialwinkelsensor ausgebildet. Die Sensoren können an dem Oberschenkelschaft 10, dem Oberteil 1, dem Unterteil 2, dem Unterschenkelrohr 5 oder dem Fußteil 3 angeordnet sein. Bei Orthesen sind die Sensoren an den jeweils korrespondierenden Schienen, Gelenkteilen oder Fußteilen befestigt, die Sensoren 9 können auch an der Gliedmaße selbst befestigt sein.

Über die Sensoren 9 wird der gesamte Schrittzyklus von dem Fersenstoß (Heel Strike) über die Zehenablösung bis zum erneuten, nachfolgenden Heel Strike HS überwacht, somit auch die gesamte Schwungphase mit der Schwungphasenextension und der Schwungphasenflexion.

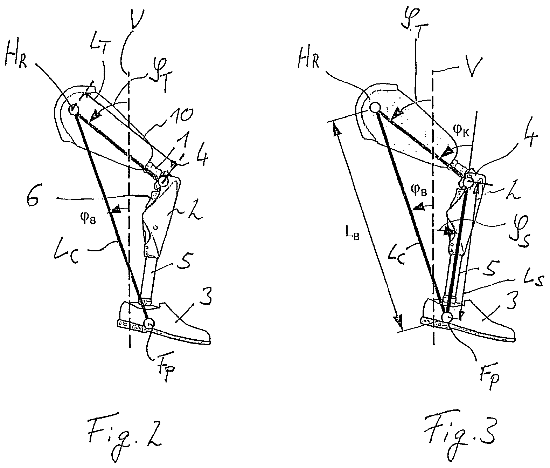

In der Figur 2 ist in einer Seitenansicht ein Prothesenkniegelenk mit dem Oberschenkelschaft 10, dem Oberteil 1, dem schwenkbar um eine Knieachse 4 angelagerten Unterteil 2 mit der darin angeordneten Widerstandseinheit 6, dem distalen Unterschenkelrohr 5 und dem daran befestigten Prothesenfuß in einer eingebeugten Stellung gezeigt. Die Beinsehne $L_C$ erstreckt sich zwischen einem Fußpunkt $F_P$ und einem Hüftdrehpunkt $H_R$. Die Beinsehne Lc ist die Verbindung zwischen dem Hüftdrehpunkt $H_R$ und dem Fußpunkt $F_P$, über die Orientierung der Beinsehne Lc lassen sich Schlüsse auf die jeweils ausgeführte Bewegung ziehen, insbesondere können unterschiedliche Bewegungen oder Gangsituationen voneinander unterschieden werden. Der Fußpunkt $F_P$ kann in der Fußmitte liegen, eine alternative Definition des Fußpunktes $F_P$ ist der Momentanpol der Polbewegung, die Schwenkachse eines Knöchelgelenkes oder die Verlängerung der Längserstreckung des Unterteils 2 auf dem Niveau der Fußsohle. Als Kenngröße für die Lage der Beinsehne Lc ist der Beinsehnenwinkel $\varphi_B$ eingezeichnet, der als Winkel zwischen der Beinsehne Lc und einer Vertikalen V definiert ist. In der dargestellten Position der Protheseneinrichtung ist das Oberteil 1 um einen Winkel relativ zum Unterteil 2 eingebeugt, die Beinsehne Lc ist somit in die Richtung nach hinten verkippt. Daraus ergibt sich, dass auch ein Oberschenkelwinkel $\varphi_T$ relativ zur Vertikalen V anliegt. Der Oberschenkelwinkel $\varphi_T$ vergrößert sich relativ zur Vertikalen V, wenn das Unterteil 2 beispielsweise senkrecht gehalten bleibt und das Oberteil 1 im dargestellten Ausführungsbeispiel entgegen dem Uhrzeigersinn um die Schwenkachse 4 verschwenkt wird. Bezugsgröße für den Oberschenkelwinkel $\varphi_T$ ist die Verbindungslinie zwischen dem Hüftdrehpunkt $H_R$ und der Knieachse 4, die Entfernung der beiden Punkte voneinander entlang dieser Verbindungslinie definiert gleichzeitig die Oberschenkellänge $L_T$.

In der Figur 3 ist zusätzlich zu den Größen gemäß Figur 2 der Kniewinkel $\varphi_K$ aufgetragen, der der Winkel zwischen dem Oberschenkelsegment, repräsentiert durch die Verbindungslinie zwischen der Knieachse 4 und dem Hüftdrehpunkt $H_R$, und der Längserstreckung des Unterteils 2 ist. Der Kniewinkel $\varphi_K$ ist 0, wenn sich die Protheseneinrichtung in einer maximal extendierten Stellung befindet. Dies bedeutet, dass die Längserstreckung des Unterteils 2 mit der Längserstreckung des Oberteils 1 fluchtet, also die Verbindung zwischen der Knieachse 4 und dem Hüftdrehpunkt $H_R$ mit der Verbindungslinie zwischen der Knieachse 4 und dem Fußpunkt $F_P$ fluchtet, wenn dieser auf der Achse der Längserstreckung des Unterschenkelrohrs 5 liegt.

Die Unterschenkellänge Ls wird definiert durch den Abstand zwischen der Knieachse 4 und dem Fußpunkt $F_P$. Der Unterschenkelwinkel $\varphi_S$ ist der Winkel zwischen der Vertikalen V und der Verbindungslinie zwischen dem Fußpunkt $F_P$ und der Knieachse 4. Im dargestellten Ausführungsbeispiel mit dem eingebeugten Prothesenkniegelenk um einen Winkel $\varphi_K$ ist der Unterschenkelwinkel $\varphi_S$ positiv in Vorwärtsgehrichtung verkippt, der Oberschenkelwinkel $\varphi_T$ ist in Rückwärtsrichtung zur Vertikalen orientiert, die Beinsehne Lc ist um den Winkel $\varphi_B$ nach hinten verkippt. Die Länge $L_B$ der Beinsehne Lc ist durch den Abstand zwischen dem Hüftdrehpunkt $H_R$ und dem Fußpunkt $F_P$ definiert.

Die Länge $L_B$ der Beinsehne Lc lässt sich aus den bekannten Segmentlängen $L_T$ und Ls in Verbindung mit dem Kniewinkel berechnen. Neben Inertialwinkelsensoren 9, die an dem Unterteil 2 oder dem Oberteil 1 bzw. dem Oberschenkelschaft 10 oder dem Unterschenkelrohr 5 angeordnet sein können, kann sich die Orientierung oder der Beinsehnenwinkel $\varphi_B$ auch aus einer Kombination des Unterschenkelwinkels $\varphi_S$ in Verbindung mit einem gewichteten Kniewinkel $\varphi_K$ abschätzen lassen, wobei die Formel hierfür lautet

$$\varphi_B = \varphi_S + d \times \varphi_K$$

, wobei d zwischen 0,4 und 0,6 liegt, insbesondere bei 0,5.

Durch die Kenntnis der Länge $L_B$ und Orientierung $\varphi_B$ der Beinsehne Lc und ggf. der zeitlichen Ableitungen dieser Größen ist es möglich, die Abrollbewegung in der Standphase unabhängig von einer Standphasenbeugung oder Standphasenstreckung zu verfolgen und Kenntnis über den Bewegungsfortschritt zu erlangen. Durch die Veränderung der Beinsehnenorientierung oder des Beinsehnenwinkels $\varphi_B$ kann der Bewegungsfortschritt sowohl in der Standphase als auch in der Schwungphase verfolgt werden, so dass diese Größe zur Steuerung des Standphasenverhaltens und/oder Schwungphasenverhaltens durch Anpassung der Dämpfereinstellungen herangezogen werden kann.

Der Oberschenkelwinkel $\varphi_T$ ebenso wie der Unterschenkelwinkel $\varphi_S$, die auch als Segmentwinkel bezeichnet werden können, können mittels Inertialsensoren gemessen werden, die sich auf dem jeweiligen Segment befinden. Alternativ erfolgt eine Berechnung über nur einen Inertialsensor auf dem jeweils nicht betroffenen Segment und dem Kniewinkel $\varphi_K$, der über einen Kniewinkelsensor ermittelt wird.

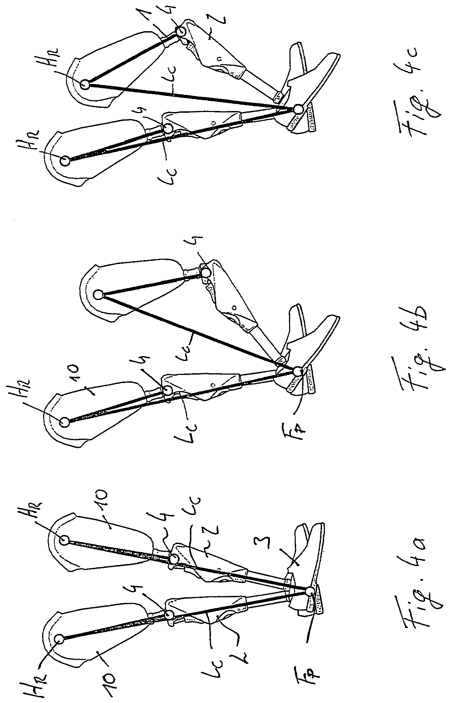

In der Figur 4a sind zwei Abschnitte einer Beinstellung für das ebene Gehen abgebildet. Die linke Darstellung zeigt die Protheseneinrichtung kurz nach dem Fersenkontakt, die Länge $L_B$ der Beinsehne Lc ist nahezu maximal, da der nicht eingezeichnete Beinsehnenwinkel $\varphi_B$ nahezu dem Oberschenkelwinkel $\varphi_S$ entspricht. Im weiteren Schrittverlauf findet eine Vorwärtsprogression statt, die Protheseneinrichtung rotiert insgesamt nach vorne, die Beinsehne Lc wird um den Fußpunkt $F_P$, der auch in einem Knöchelgelenk liegen kann, nach vorne verschwenkt, so dass die Beinsehne Lc vor der Vertikalen liegt. Der Figur 4a ist zu entnehmen, dass sich über den großen Bereich der Vorwärtsprogression beim Gehen in der Ebene der Oberschenkel bzw. Oberschenkelschaft 10 zusammen mit der Beinsehne Lc bei nahezu gestrecktem Kniegelenk mit nach vorne verlagert, eine Veränderung des Kniewinkels findet nicht statt.

In der Figur 4b ist das Gehen auf einer abwärts gerichteten Rampe gezeigt. In der linken Darstellung ist eine vergrößerte Beugung im Prothesenkniegelenk zu erkennen, der Oberschenkelschaft 10 ist um die Knieachse 4 verschwenkt, die Orientierung der Beinsehne Lc entspricht ungefähr der in der Aufsetzphase beim Gehen in der Ebene.

Der weitere Verlauf der Bewegung beim Rampe abwärtsgehen, ist in der rechten Darstellung der Figur 4b gezeigt. Ebenfalls erfolgt eine Abrollbewegung um den Fußpunkt Fp, die Beinsehne $L_C$ rotiert um den Fußpunkt Fp nach vorne, der Oberschenkelwinkel $\varphi_S$ bleibt aufgrund einer vergrößerten Einbeugung um die Knieachse 4 in einer nahezu konstanten Position, d. h., dass sich die Orientierung des Oberschenkels oder Oberschenkelschaftes 10 im Raum nicht oder nur unwesentlich ändert, während die Beinsehne Lc eine Vorwärtsrotation ausführt.

Eine dritte Gangsituation ist in der Figur 4c dargestellt, nämlich das Treppabgehen. Die Position der einzelnen Komponenten der Protheseneinrichtung entspricht in der Anfangsstellung, die in der linken Darstellung gezeigt ist, das Rampe abwärtsgehen. Die Beinsehne Lc ist nach hinten geneigt, also entgegen dem Uhrzeigersinn zur Vertikalen nach hinten verkippt. Im weiteren Verlauf des alternierenden Treppabgehens wird die Protheseneinrichtung eingebeugt, eine Verschwenkung des Oberteils 1 zu dem Unterteil 2 findet statt, der Kniewinkel $\varphi_K$ vergrößert sich, ebenso verringert sich die Länge $L_P$ der Beinsehne Lc. Die Orientierung der Beinsehne Lc ändert sich weniger als beim Gehen in der Ebene oder beim Rampe abwärtsgehen, d. h., dass eine Vorwärtsrotation der Beinsehne Lc nur in einem geringeren Maße stattfindet, der Beinsehnenwinkel $\varphi_B$ zu der Vertikalen somit kleiner als beim Gehen in der Ebene oder beim Rampe abwärtsgehen ist.

Bei den Gangsituationen gemäß der Figuren 4a bis 4c findet Vorwärtsprogression statt, das Bein rollt also nach vorne ab. Ein geeigneter Parameter oder eine Hilfsvariable zur Unterscheidung und Erkennung der jeweiligen Gangsituation ist der Quotient zwischen der Änderung des Oberschenkelwinkels $\varphi_T$ oder des Unterschenkelwinkels $\varphi_S$ und der Änderung der Beinorientierung oder des Beinsehnenwinkels $\varphi_B$. Ebenso sind die zeitlichen Ableitungen als Kenngröße geeignet und vorgesehen, also der Quotient der Änderung der Winkelgeschwindigkeiten der Beinsehne Lc und des Oberschenkels bzw. des Unterschenkels.

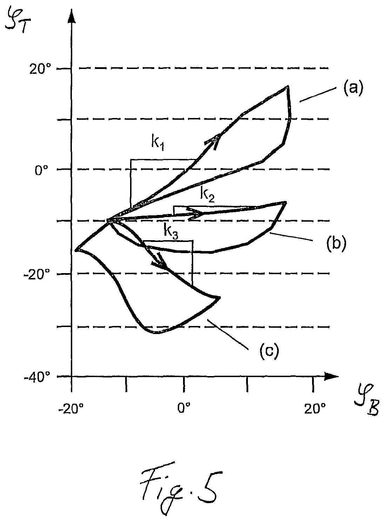

In der Figur 5 sind die Verläufe der jeweiligen Winkel aufgetragen, in der Kurve a ist der Verlauf der Winkel für das ebene Gehen gezeigt, in der Kurve b der Verlauf der Beinsehnen- und Oberschenkelwinkel für das Rampe abwärtsgehen, in der Kurve c der Verlauf der Winkel für das Treppabgehen.

Es wird deutlich, dass alle Kurven a, b, c einen unterschiedlichen Verlauf haben, insbesondere ist die Steigung k für den jeweiligen Kurvenverlauf verschieden. Die Steigung k kann als Differenzenquotient bestimmt werden, die Formel hierzu lautet

$$k = (\varphi_{T1} - \varphi_{T0}) / (\varphi_{B1} - \varphi_{B0})$$

Für das Gehen in der Ebene ist die Steigung $k_1$ wesentlich größer als die Steigung $k_2$ für das Rampe abwärtsgehen. Während sich beim Gehen in der Ebene gemäß Kurve a die Änderung des Oberschenkelwinkels $\varphi_T$ im Wesentlichen an der Änderung des Beinsehnenwinkels $\varphi_B$ orientiert, die Steigung annähernd 1 ist, ist der Oberschenkelwinkel $\varphi_T$ beim Rampe Abwärtsgehen nahezu konstant, so dass sich eine wesentlich geringere Steigung $k_2$ für das Rampe Abwärtsgehen einstellt. Beim Treppabgehen verringert sich der Beinsehnenwinkel $\varphi_B$ wesentlich geringer als der Oberschenkelwinkel $\varphi_T$, so dass für die Steigung $k_3$ beim Treppabgehen sich ein negativer Wert einstellt.

In Abhängigkeit von dem detektierten Quotienten oder der jeweiligen Steigung $k_1$, $k_2$, $k_3$ kann eine Anpassung der Widerstände erfolgen, die Standardeinstellung für das Gehen in der Ebene kann beim Detektieren des Rampe Abwärtsgehens gemäß Kurvenverlauf b dahingehend verändert werden, dass ein Nachgeben und damit eine verringerte Flexion bei einem entsprechenden Beinsehnenwinkel $\varphi_B$ vorliegt. Wird eine negative Steigung $k_3$ gemäß Kurve c in Figur 5 detektiert, kann das Treppabgehen angenommen werden. Ein langsames Einsinken oder das Vermeiden einer kompletten Sperrung der Protheseneinrichtung sollte vermieden werden, um einen Katapulteffekt auszuschließen.

Entsprechend charakteristische Phasendiagramme werden erhalten, wenn statt der Winkel die Winkelgeschwindigkeiten oder die Winkelbeschleunigungen von Beinsehne und Oberschenkel oder Unterschenkel aufgetragen werden.

Mit dem erfindungsgemäßen Verfahren müssen keine Kräfte oder Kraftverläufe gemessen oder ausgewertet werden, um eine Unterscheidung von Gangsituationen und deren Bewegungsfortschritt zu erfassen. Es werden grundsätzlich nur Winkel gemessen, errechnet oder abgeschätzt und der Veränderung der Dämpfereinstellung zugrunde gelegt.

**Patentansprüche**

1. Verfahren zur Steuerung einer Widerstandsänderung bei einem künstlichen Gelenk einer Orthese, eines Exoskelettes oder Prothese einer unteren Extremität, wobei das künstliche Gelenk ein Oberteil (1) und ein Unterteil (2) aufweist, die um eine Schwenkachse (4) schwenkbar aneinander befestigt sind, wobei eine Widerstandseinheit (6) zwischen dem Oberteil (1) und dem Unterteil (2) befestigt ist, um einen Widerstand gegen eine Einbeugung oder Streckung des künstlichen Gelenks bereitzustellen und der Widerstandseinheit (6) eine Verstelleinrichtung (7) zugeordnet ist, über die der Widerstand verändert wird, wenn ein Sensorsignal einer der Verstelleinrichtung (7) zugeordneten Steuereinheit (8) die Verstelleinrichtung (7) aktiviert, **dadurch gekennzeichnet, dass** der Widerstand in Abhängigkeit von der Lage ($\varphi_B$) und/oder Länge ($L_B$) der gemessenen oder berechneten Beinsehne (Lc) und/oder deren zeitlicher Ableitungen verändert wird, wobei als Beinsehne (Lc) die Verbindungslinie zwischen einem Hüftdrehpunkt ($H_R$) und einem Fußpunkt (Fp) verwendet wird.

2. Verfahren nach Anspruch 1, **dadurch gekennzeichnet, dass** die Lage ($\varphi_B$) der Beinsehne (Lc) als Summe aus einem Unterschenkelwinkel ($\varphi_S$) und einem Bruchteil eines Kniewinkels ($\varphi_K$) abgeschätzt oder über den Unterschenkelwinkel ($\varphi_S$), den Kniewinkel ($\varphi_K$) und die Oberschenkelsegmentlänge ($L_T$) und die Unterschenkelsegmentlänge (Ls) errechnet wird.

3. Verfahren nach Anspruch 1 oder 2, **dadurch gekennzeichnet, dass** der Unterschenkelwinkel ($\varphi_S$) oder Oberschenkelwinkel ($\varphi_T$) über einen Inertialwinkelsensor (9) direkt gemessen oder über einen Lagesensor (9) an einem Oberschenkel (1, 10) oder Unterschenkel (2, 5) und einen Kniewinkelsensor (9) ermittelt wird.

4. Verfahren nach einem der voranstehenden Ansprüche, **dadurch gekennzeichnet, dass** die Länge ($L_B$) der Beinsehne (Lc) aus dem Kniewinkel ($\varphi_K$) und den Segmentlängen von Oberschenkel ($L_T$) und Unterschenkel (Ls) bestimmt wird.

5. Verfahren nach einem der voranstehenden Ansprüche, **dadurch gekennzeichnet, dass** der Widerstand in Abhängigkeit von der Richtung der Veränderung der Lage ($\varphi_B$) und/oder Länge ($L_B$) der Beinsehne (Lc) verändert wird.

6. Verfahren nach einem der voranstehenden Ansprüche, **dadurch gekennzeichnet, dass** ein Quotient (k) aus der Änderung der Lage ($\varphi_B$) der Beinsehne und der Änderung des Oberschenkelwinkels ($\varphi_T$) oder Unterschenkelwinkel ($\varphi_S$) ermittelt und zur Beurteilung der Gangsituation verwendet wird.

7. Verfahren nach einem der voranstehenden Ansprüche, **dadurch gekennzeichnet, dass** ein Quotient (k) aus der Änderung der Beinsehnengeschwindigkeit und der Änderung der Oberschenkelgeschwindigkeit ($\varphi_T$) oder Unterschenkelgeschwindigkeit ($\varphi_S$) ermittelt und zur Beurteilung der Gangsituation verwendet wird.

8. Verfahren nach einem der voranstehenden Ansprüche, **dadurch gekennzeichnet, dass** zur Detektion einer Standphase oder des Stehens ein Kraftsensor (9) zur Erfassung von Kräften in dem Unterteil (2) verwendet wird.

9. Verfahren nach einem der voranstehenden Ansprüche, **dadurch gekennzeichnet, dass** der Widerstand zusätzlich in Abhängigkeit von der Lage oder der Lageänderung des Oberteils (1) und / oder des Unterteils (2) verändert wird.

10. Verfahren nach einem der voranstehenden Ansprüche, **dadurch gekennzeichnet, dass** der Widerstand insbesondere geändert wird, wenn die Lage und / oder die Lageänderung der Beinsehne (Lc) einen vorbestimmten Grenzwert überschreitet oder unterschreitet.

**Claims**

1. A method for controlling a change in resistance in an artificial joint of an orthosis, of an exoskeleton or prosthesis of a lower extremity, wherein the artificial joint has an upper part (1) and a lower part (2) which are fastened to one another pivotably about a pivot axis (4), wherein a resistance unit (6) is fastened between the upper part (1) and the lower part (2) in order to provide a resistance to flexion or extension of the artificial joint, and the resistance unit (6) is assigned an adjustment device (7) by means of which the resistance is changed if a sensor signal of a control unit (8) assigned to the adjustment device (7) activates the adjustment device (7), **characterized in that** the resistance is changed in a manner dependent on the position ($\varphi_B$) and/or length ($L_B$) of the measured or calculated leg chord (Lc) and/or the derivatives thereof with respect to time, wherein the connecting line between a hip center of rotation ($H_R$) and a foot point ($F_P$) is used as leg chord (Lc).

2. The method as claimed in claim 1, **characterized in that in that** the position ($\varphi_B$) of the leg chord (Lc) is estimated as a sum of a lower leg angle ($\varphi_S$) and a fraction of a knee angle ($\varphi_K$), or is calculated using the lower leg angle ($\varphi_S$), the knee angle ($\varphi_K$) and the thigh segment length ($L_T$) and the lower leg segment length (Ls).

3. The method as claimed in claim 1 or 2, **character-**

**ized in that** the lower leg angle ($\varphi_S$) or thigh angle ($\varphi_T$) is directly measured by means of an inertial angle sensor (9), or is determined by means of an position sensor (9) on a thigh (1, 10) or lower leg (2, 5) and a knee angle sensor (9).

4. The method as claimed in one of the preceding claims, **characterized in that** the length ($L_B$) of the leg chord (Lc) is determined from the knee angle ($\varphi K$) and the segment lengths of thigh ($L_T$) and lower leg (Ls).

5. The method as claimed in one of the preceding claims, **characterized in that** the resistance is changed in a manner dependent on the direction of the change in the position ($\varphi_B$) and/or length ($L_B$) of the leg chord (Lc).

6. The method as claimed in one of the preceding claims, **characterized in that** a quotient (k) is determined from the change in the position ($\varphi_B$) of the leg chord and the change in the thigh angle ($\varphi_T$) or lower leg angle ($\varphi S$) and is used for the assessment of the walking situation.

7. The method as claimed in one of the preceding claims, **characterized in that** a quotient (k) is determined from the change in the leg chord speed and the change in the thigh speed ($\varphi_T$) or a lower leg speed ($\varphi S$) and is used for the assessment of the walking situation.

8. The method as claimed in one of the preceding claims, **characterized in that** a force sensor (9) for detecting forces in the lower part (2) is used for the detection of a stance phase or of standing.

9. The method as claimed in one of the preceding claims, **characterized in that** the resistance is changed additionally in a manner dependent on the position or change in position of the upper part (1) and/or of the lower part (2).

10. The method as claimed in one of the preceding claims, **characterized in that** the resistance is changed in particular if the position and/or the change in position of the leg chord (Lc) overshoots or undershoots a predetermined threshold value.

## Revendications

1. Procédé pour commander une modification de la résistance dans une articulation artificielle d'une orthèse, d'un exosquelette ou d'une prothèse d'un membre inférieur, l'articulation artificielle comprenant une partie supérieure (1) et une partie inférieure (2) qui sont fixées l'une à l'autre de manière à pouvoir pivoter autour d'un axe de pivotement (4), une unité de résistance (6) étant fixée entre la partie supérieure (1) et la partie inférieure (2) pour fournir une résistance à la flexion ou à l'extension de l'articulation artificielle, et un dispositif de réglage (7) étant associé à l'unité de résistance (6), par l'intermédiaire duquel la résistance est modifiée lorsqu'un signal de capteur d'une unité de commande (8) associée au dispositif de réglage (7) vient activer le dispositif de réglage (7), **caractérisé en ce que** la résistance est modifiée en fonction de la position ($\varphi B$) et/ou de la longueur ($L_B$) du tendon de la jambe ($L_C$) mesuré ou calculé et/ou en fonction de ses dérivées temporelles, en utilisant comme tendon de la jambe ($L_C$) la ligne de liaison entre un point de rotation de la hanche ($H_R$) et un point de pied ($F_P$).

2. Procédé selon la revendication 1, **caractérisé en ce que** la position ($\varphi B$) du tendon de la jambe ($L_C$) est estimée comme la somme d'un angle du bas de jambe ($\varphi_S$) et d'une fraction d'un angle du genou ($\varphi_K$), ou est calculée par l'intermédiaire de l'angle du bas de jambe ($\varphi_S$), de l'angle du genou ($\varphi_K$) et de la longueur du segment de la cuisse ($L_T$) et de la longueur du segment du bas de jambe (Ls).

3. Procédé selon la revendication 1 ou 2, **caractérisé en ce que** l'angle du bas de jambe ($\varphi_S$) ou l'angle de la cuisse ($\varphi_T$) est mesuré directement par un capteur d'angle inertiel (9) ou déterminé par un capteur de position (9) sur une cuisse (1, 10) ou sur un bas de jambe (2, 5) et par un capteur d'angle de genou (9).

4. Procédé selon l'une des revendications précédentes, **caractérisé en ce que** la longueur ($L_B$) du tendon de la jambe ($L_C$) est déterminée à partir de l'angle du genou ($\varphi_K$) et des longueurs des segments de la cuisse ($L_T$) et du bas de jambe (Ls).

5. Procédé selon l'une des revendications précédentes, **caractérisé en ce que** la résistance est modifiée en fonction du sens de la modification de la position ($\varphi B$) et/ou de la longueur ($L_B$) du tendon de la jambe ($L_C$).

6. Procédé selon l'une des revendications précédentes, **caractérisé en ce qu'**un quotient (k) de la modification de la position ($\varphi B$) du tendon de la jambe et de la modification de l'angle de la cuisse ($\varphi_T$) ou l'angle du bas de jambe ($\varphi_S$) est déterminé et utilisé pour évaluer la situation de la marche.

7. Procédé selon l'une des revendications précédentes,
**caractérisé en ce qu'**un quotient (k) de la modification de la vitesse du tendon de la jambe et de la modification de la vitesse de la cuisse ($\varphi_T$) ou de la vitesse du bas de jambe ($\varphi_S$) est déterminé et utilisé pour évaluer la situation de la marche.

8. Procédé selon l'une des revendications précédentes,
**caractérisé en ce que**, pour détecter une phase debout ou la posture debout, on utilise un capteur de force (9) pour détecter des forces dans la partie inférieure (2).

9. Procédé selon l'une des revendications précédentes,
**caractérisé en ce que** la résistance est modifiée en supplément en fonction de la position ou de la modification de la position de la partie supérieure (1) et/ou de la partie inférieure (2).

10. Procédé selon l'une des revendications précédentes,
**caractérisé en ce que** la résistance est modifiée en particulier lorsque la position et/ou la modification de la position du tendon de la jambe ($L_C$) passe au-dessus ou au-dessous d'une valeur limite prédéterminée.

Fig. 1

Fig. 3

Fig. 2

12

Fig. 4a          Fig. 4b          Fig. 4c

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008008284 A1
- DE 102006021802 A1
- DE 102009052887 A1
- DE 102012003369 A1